# EUROPÄISCHE PATENTSCHRIFT

(11) **EP 0 630 885 B1**
(45) Veröffentlichungstag und Bekanntmachung des Hinweises auf die Patenterteilung: **06.12.1995**
(21) Anmeldenummer: 93109817.2
(22) Anmeldetag: 19.06.1993
(51) Int. Cl.: C07C 253/30, C07C 255/24

(54) **Verfahren zur Herstellung von Aminopropionitrilen**
Process for the preparation of aminopropionitriles
Procédé pour la préparation d'aminopropionitriles

(43) Veröffentlichungstag der Anmeldung: 28.12.1994
(73) Patentinhaber: BASF Aktiengesellschaft, 67063 Ludwigshafen (DE)
(72) Erfinder: Merger, Franz, Dr., D-6710 Frankenthal (DE); Brudermueller, Martin, Dr., D-6800 Mannheim 1 (DE); Priester, Claus-Ulrich, Dr., D-6700 Ludwigshafen (DE); Harder, Wolfgang, Dr., D-6940 Weinheim (DE); Winderl, Siegfried, Dr., D-6900 Heidelberg (DE)

(56) Entgegenhaltungen:
- US-A- 1 992 615
- CHEMICAL ABSTRACTS, vol. 69, no. 19, 4. November 1968, Columbus, Ohio, US; abstract no. 76587s, MUROHASHI, SUSUMU 'Amination of acrylonitrile in liquid ammonia'
- CHEMICAL ABSTRACTS, vol. 74, no. 23, 7. Juni 1971, Columbus, Ohio, US; abstract no. 124903u, MUROHASHI, SUSUMU '.beta.-Aminopropionitrile'
- ORGANIC REACTIONS Bd. V , 1949 Seiten 79 - 135 H.A. BRUSON 'Cyanoethylation'

## Beschreibung

Die vorliegende Erfindung betrifft ein neues und verbessertes Verfahren zur Herstellung von Aminopropionitrilen durch Umsetzung von überschüssigem Ammoniak mit Acrylnitrilen an Heterogenkatalysatoren bei erhöhten Temperaturen und erhöhten Drücken.

Aus J. Chem. Soc., 1369 bis 1371 (1947) ist die Umsetzung von ω-Bromalkylcyaniden mit dem Kaliumsalz des Phthalsäureimids und nachfolgende Reaktion mit Hydrazinhydrat beschrieben. Dieses Verfahren ist z.B. zur technischen Herstellung von 3-Aminopropionsäurenitril aber ungeeignet.

Aus der US-A 3 174 992 ist die Umsetzung von Ethylencyanhydrin mit Anmoniak an feuchtem Raney-Nickel bei Temperaturen um 100°C unter Eigendruck bekannt. Die Reaktion verläuft mit 54 % Ausbeute unbefriedigend.

3-Aminopropionsäurenitril und Bis-(2-cyanethyl)amin erhält man im ungünstigen Verhältnis 2/1 auch auf dem technisch aufwendigen Umweg über 2-Methoxypropionsäurenitril durch Umsetzung mit Ammoniak in Gegenwart von Raney-Cobalt bei Temperaturen von 65 bis 80°C und 165 bar Druck nach DE-A-10 03 740.

Zur Herstellung von 3-Aminopropionsäurenitril durch Direktumsetzung von Acrylnitril mit Ammoniak ist bekannt, daß wasserfreies Ammoniak bei Raumtemperatur nicht mit Acrylnitril reagiert, sondern vielmehr als Stabilisator für Acrylnitril eingesetzt werden kann (US-A 2 432 511). Aus der US-A 2 401 429 ist bekannt, daß man nach 2 Tagen aus Acrylnitril und flüssigem Ammoniak bei Raumtemperatur neben 76 % Bis-(2-cyanethyl)ether auch 11 % 3-Aminopropionsäurenitril isolieren kann. Bei 90°C setzt sich Acrylnitril unter Druck mit flüssigem Ammoniak zu 12,5 % 3-Aminopropionitril um (DE-A-598 185).

Ferner ist bekannt, daß sich der Zusatz von protischen Lösungsmitteln vorteilhaft auf die Addition von NH₃ an Acrylnitril auswirkt. Der Zusatz von Wasserdampf zum Gemisch aus Acrylnitril-Ammoniak ist beispielsweise aus der Chem. Abstr. Vol. 83, 26879 bekannt. Üblicherweise wird jedoch wäßriger Ammoniak im Temperaturbereich von 80 bis 130°C eingesetzt. Bei einem Verhältnis von Ammoniak/Acrylnitril/Wasser 5 bis 15 : 1 : 5 bis 20 erhält man 3-Aminopropionitril neben Bis-(2-cyanethyl)amin in Ausbeuten von 57 bis 80 % (z.B. US-A 3 935 256 -62 %, DE-A-24 36 651 -70 %, USA 2 448 013 -78 %, US-A-2 019 903 -59 %, Org. Syn. 27, 3 bis 5 (1947) -57 %). Nachteile dieser Verfahren unter Einsatz von wäßrigem Ammoniak ergeben sich bei der Auf- bzw. Weiterverarbeitung des Produktgemisches:
- Destillative Entfernung von zugesetztem Wasser bei der in Folge großer Ammoniakmengen nötigen Kreislauffahrweise
- Selektivitätsverluste für 3-Aminopropionsäurenitril bei der Abtrennung von Ammoniak/Wasser
- Hydrolyse der Nitrilgruppen
- Katalysatorschädigung bei der nachfolgenden Hydrierung.

Chem. Abstr., Vol 69, 75 587 (1968) beschreibt ein Verfahren zur Herstellung von Aminopropionitril durch Umserzung von Acrylnitril mit Ammoniak, im Molverhältnis von 1 : (>10), an Heterogenkatalysatoren (Raney Cu oder Co) bei 100°C.

Aus US-A-1 992 615 ist bekannt, schwach saure Katalysatoren für die Herstellung von Aminopropionitrilen aus Acrylnitril und Ammoniak zu verwenden.

Aus Przemyst. Chem. 44(2), 85 (1965), bzw. GB-A-642 409 sind Verfahren bekannt, bei denen durch Zusatz von 15 bis 20 Äquivalenten Methanol eine Ausbeute von 81 % 3-Aminopropionitril erzielt werden. Die Bildung von Nebenprodukten durch Methylierung wirkt prohibitiv für die technische Nutzung des Verfahrens. Eine Ausbeute von 68 % 3-Aminopropionitril erhält man hingegen bei Zusatz von 3 Äquivalenten tert.-Butanol (US-A 2 742 491).

Die in DE-A-24 36 651 beschriebene Herstellung von 3-Aminopropionitril durch Aminolyse von Bis-(2-cyanethyl)amin mit Ammoniak verlangt Temperaturen von 130 bis 170°C und verläuft sehr langsam mit Reaktionszeiten von bis zu 165 min.

Der vorliegenden Erfindung lag daher die Aufgabe zugrunde, den zuvor genannten Nachteilen abzuhelfen.

Demgemäß wurde ein neues und verbessertes Verfahren zur Herstellung von Aminopropionitrilen der allgemeinen Formel I 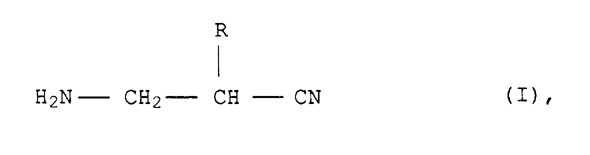
R Wasserstoff oder Methyl bedeutet, welches dadurch gekennzeichnet ist, daß man Ammoniak mit Acrylnitrilen der allgemeinen Formel II 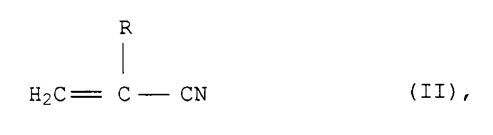
in der die Substituenten die oben genannten Bedeutungen haben, im Molverhältnis von 1:1 bis 500:1 an Heterogenkatalysatoren der Oxide der zweiten und/oder dritten und/oder vierten Hauptgruppe, der zweiten bis sechsten Nebengruppe des Periodensystems der Elemente, saure Zeolithe, Phosphate und/oder Heteropolysäuren oder Gemische aus diesen, bei Temperaturen von 40 bis 180°C und Drücken von 10 bis 350 bar umsetzt.

Das erfindungsgemäße Verfahren läßt sich wie folgt durchführen:
Die Reaktion kann bei Temperaturen von 40 bis 180°C und Drücken von 10 bis 350 bar diskontinuierlich oder vorzugsweise kontinuierlich durchgeführt werden. Das bevorzugte kontinuierliche Verfahren kann bei Temperaturen von 40 bis 150°C und Drücken von 10 bis 200 bar, vorzugsweise bei 50 bis 120°C und 150 bis 230 bar durchgeführt werden.

Üblicherweise wird Ammoniak mit 0 bis 5 Gew.-% Wasser, bevorzugt im wesentlichen wasserfrei (0 bis 1 Gew.-% Wasser) besonders bevorzugt mit 0,1 - 1 Gew.-% Wasser mit den Acrylnitrilen im Molverhältnis von 1:1 bis 500:1, bevorzugt 2:1 bis 100:1, besonders bevorzugt 10:1 bis 80:1 eingesetzt. In der Reaktion nichtumgesetzter Ammoniak kann ohne Verschlechterung der Aminopropionitril-Ausbeute technisch einfach in die Umsetzung zurückgeführt werden (Kreislauffahrweise).

Im allgemeinen verwendet man keine Lösungsmittel; man kann jedoch gegebenenfalls inerte Lösungsmittel z.B. Ether wie Dibutylether, Tetrahydrofuran, Dimethoxiether, oder z.B. Kohlenwasserstoffe wie Cyclohexan, Benzol und Toluol in Mengen von 0 bis 500 Gew.%, bevorzugt 50 bis 200 Gew.-% zum Acrylnitril II einsetzen.

Bei der Umsetzung hält man vorteilhaft eine Katalysatorbelastung von 0,1 bis 10 g, vorzugsweise 0,1 bis 2 g Acrylnitril je g Katalysator und Stunde ein.

Als Heterogenkatalysatoren eignen sich insbesondere sauer und/oder basisch bzw. amphoter wirkende Oxide von Elementen der zweiten, dritten oder vierten Hauptgruppe, insbesondere verschiedene Modifikationen von Al₂O₃ und SiO₂ in Form von Kieselgel, Kieselgur, Quarz oder Gemischen aus diesen, sowie der zweiten bis sechsten Nebengruppe des Periodensystems bzw. Gemische aus diesen. Als weiterhin vorteilhafte Katalysatoren sind Zinkoxid, Titanoxid, Zirkonoxid, Vanadiumoxide, Nioboxid, Boroxid, Chromoxide, Molybdänoxide, Wolframoxide oder Gemische dieser zu nennen. Auch Gemische dieser Oxide mit Aluminiumoxid eignen sich für diese Reaktion. Weitere Katalysatoren für das erfindungsgemäße Verfahren sind Zeolithe, Phosphate, oder Heteropolysäuren.

Als Heterogenkatalysatoren eignen sich ferner Lanthanoxid und/oder Oxide der Lanthaniden wie Ceroxid, Praseodymoxid und Neodymoxid bevorzugt Ceroxid oder Gemische dieser mit sauer und/oder basisch bzw. amphoter wirkenden Oxiden von Elementen der zweiten, dritten und vierten Hauptgruppe des Periodensystems, insbesondere verschiedene Modifikationen von Al₂O₃ und SiO₂ in Form von Kieselgel, Kieselgur oder Gemischen aus diesen.

Die in diesem Verfahren verwendeten Aminopropionitrile der allgemeinen Formel I sind:
3-Aminopropionsäurenitril,
3-Amino-isobutyronitril.

Die nach dem erfindungsgemäßen Verfahren herstellbaren Aminopropionitrile der allgemeinen Formel I sind Zwischenprodukte für die Herstellung von Diamine, Aminocarbonsäuren bzw. Aminocarbonsäureamide.

Das nach dem erfindungsgemäßen Verfahren herstellbare 3-Aminopropionitril der allgemeinen Formel I eignet sich als Zwischenprodukt für die Herstellung von
- β-Aminosäuren: β-Alanin --->Vorprodukt für Calciumpanthothenat durch Hydrolyse (DE-A-22 23 236)
- 1,3-Propylendiamin, die Verwendung in Pharmazeutika, Polyamiden und Holzschutzmitteln finden (DE-A 32 48 326, DT 20 04 405)

### Beispiele

### Beispiel 1

Durch einen mit 19,8 g SiO₂ (1 bis 3 mm Splitt) gefüllten Rohrreaktor wurde bei 90°C und einem Druck von 180 bar ein Gemisch aus 270 ml flüssigem Ammoniak und 20 ml Acrylnitril (Belastung 0,8 g Acrylnitril g⁻¹ Kat. h⁻¹) gepumpt. Der Umsatz betrug 99 %. Nach 100 Stunden Betriebsdauer wies der Reaktionsaustrag folgende Zusammensetzung auf (quant. GC):
85,1 Gew.-% 3-Aminopropionitril
13,0 Gew.-% Bis-(2-cyanethyl)amin
0,9 Gew.-% Acrylnitril

### Beispiel 2

Durch einen mit 20,7 g Borzeolith ZBM-11 (SiO₂/B₂O₃ = 33,9, 2,5 mm Stränge) gefüllten Rohrreaktor wurde bei 130°C und einem Druck von 180 bar ein Gemisch aus 280 ml flüssiger Ammoniak und 21 ml Acrylnitril (Belastung 0,8 g Acrylnitril g⁻¹ Katalysator h⁻¹) gepumpt. Nach 4 Betriebsstunden wurde ein Umsatz von 94 % und ein Reaktionsaustrag mit folgender Zusammensetzung (quant. GC) erhalten:
84,7 Gew.-% 3-Aminopropionitril
14,3 Gew.-% Bis-(2-cyanethyl)amin
3,0 Gew.-% Acrylnitril

### Beispiel 3

Über 28 g Zeolith ZSM-11 (SiO₂/Al₂O₃ = 145,7, 2,5 mm Stränge), angeordnet in einem Rohrreaktor, werden bei 110°C und einem Druck von 180 bar 275 ml flüssiger Ammoniak und 20 ml Acrylnitril (Belastung 0,57 g Acrylnitril g⁻¹ Katalysator h⁻¹) gepumpt. Nach 4 Betriebsstunden wird ein Reaktionsaustrag mit folgender Zusammensetzung (quant. GC) isoliert (Umsatz 97 %):
80,0 Gew.-% 3-Aminopropionitril
14,9 Gew.-% Bis-(2-cyanethyl)amin
3,1 Gew.-% Acrylnitril

### Beispiel 4

280 ml flüssiger Ammoniak und 20 ml Acrylnitril werden über 33 g in einen Rohrreaktor gefülltes Aluminiumoxid/Siliciumoxid 8/2 (2 bis 3 mm Gries) (Belastung: 0,48 g Acrylnitril g⁻¹ h⁻¹) geleitet. Bei 90°C und einem Druck von 180 bar erhält der Reaktionsaustrag nach einer Betriebsdauer von 48 Stunden bei einem Umsatz von 100 % folgende Zusammensetzung (quant. GC):
90,6 Gew.-% 3-Aminopropionitril
9,4 Gew.-% Bis-(2-cyanethyl)amin

### Beispiel 5

Über 33 g Aluminiumoxid (1 bis 33 mm Gries), das in einen Rohrreaktor eingefüllt ist, werden 290 ml flüssiger Ammoniak und 20 ml Acrylnitril (Belastung 0,48 g Acrylnitril g⁻¹ h⁻¹) bei 55°C und einem Druck von 180 bar gepumpt. Nach 400 Betriebsstunden wird ein Umsatz von 97 % erreicht und ein Reaktionsaustrag mit folgender Zusammensetzung (quant. GC) isoliert:
77,6 Gew.-% 3-Aminopropionitril
20,2 Gew.-% Bis-(2-cyanethyl)amin
2,2 Gew.-% Acrylnitril

### Beispiel 6

Durch einen mit 125 g Ceroxid (1 bis 2 mm Splitt) gefüllten Rohrreaktor wurde bei 90°C und einem Druck von 200 bar ein Gemisch aus 275 ml/h flüssigem Ammoniak und 20,7 ml/h Acrylnitril (Belastung 0,13 g Acrylnitril g⁻¹ Kat. h⁻¹) gepumpt. Der Umsatz betrug 92 %. Nach 50 Stunden Betriebsdauer wies der Reaktionsaustrag folgende Zusammensetzung auf (quant.GC):
59,1 Gew.-% 3-Aminopropionitril
30,6 Gew.-% Bis-(2-cyanethyl)amin
10,3 Gew.-% Acrylnitril

### Beispiel 7

Durch einen mit 35,5 g Katalysator Aluminiumoxid/Ceroxid (70 Gew.-%/30 Gew.-%; 1 bis 2 mm Splitt) gefüllten Rohrreaktor wurde bei 50°C und einem Druck von 200 bar ein Gemisch aus 276,6 ml/h flüssiger Ammoniak und 21,5 ml/h Acrylnitril (Belastung 0,49 g Acrylnitril g⁻¹ Katalysator h⁻¹) gepumpt. Der Umsatz betrug > 99 %. Nach 70 Stunden Betriebsdauer wies der Reaktionsaustrag folgende Zusammensetzung auf (quant. GC):
77,0 Gew.-% 3-Aminopropionitril
22,4 Gew.-% Bis-(2-cyanethyl)amin
0,7 Gew.-% Acrylnitril

### Beispiel 8

Durch einen mit 35,5 g Katalysator Aluminiumoxid/Ceroxid (70 Gew.-%/30 Gew.-%; 1 bis 2 mm Splitt) gefüllten Rohrreaktor wurde bei 70°C und einem Druck von 200 bar ein Gemisch aus 273,3 ml/h flüssigem Ammoniak und 16,5 ml/h Acrylnitril (Belastung 0,38 g Acrylnitril g⁻¹ Kat. h⁻¹) gepumpt. Der Umsatz betrug > 99 %. Nach 30 Stunden Betriebsdauer wies der Reaktionsaustrag folgende Zusammensetzung auf (quant. GC):
85,4 Gew.-% 3-Aminopropionitril
14,0 Gew.-% Bis-(2-cyanethyl)amin
0,6 Gew.-% Acrylnitril

## Patentansprüche

1. Verfahren zur Herstellung von Aminopropionitrilen der allgemeinen Formel I 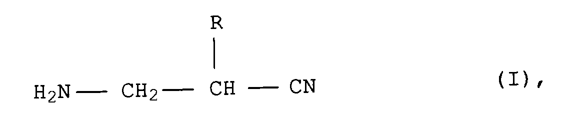 in der
R Wasserstoff oder Methyl
bedeutet, dadurch gekennzeichnet, daß man Ammoniak mit Acrylnitrilen der allgemeinen Formel II 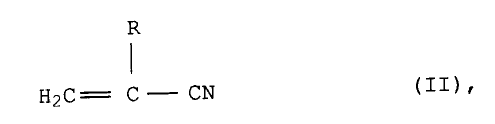 in der die Substituenten die oben genannten Bedeutungen haben, im Molverhältnis von 1:1 bis 500:1 an Heterogenkatalysatoren der Oxide der zweiten und/oder dritten und/oder vierten Hauptgruppe, der zweiten bis sechsten Nebengruppe des Periodensystems der Elemente, saure Zeolithe, Phosphate und/oder Heteropolysäuren oder Gemische aus diesen, bei Temperaturen von 40 bis 180°C und Drücken von 10 bis 350 bar umsetzt.

2. Verfahren zur Herstellung von Aminopropionitrilen nach Anspruch 1, dadurch gekennzeichnet, daß R für Wasserstoff steht.

3. Verfahren zur Herstellung von Aminopropionitrilen nach Anspruch 1, dadurch gekennzeichnet, daß man als Heterogenkatalysatoren saure Heterogenkatalysatoren verwendet.

4. Verfahren zur Herstellung von Aminopropionitrilen nach Anspruch 1, dadurch gekennzeichnet, daß man Ammoniak mit den Acrylnitrilen II im Molverhältnis von 10:1 bis 80:1 einsetzt.

5. Verfahren zur Herstellung von Aminopropionitrilen nach Anspruch 1, dadurch gekennzeichnet, daß man die Umsetzung bei Temperaturen von 50 bis 120°C durchführt.

6. Verfahren zur Herstellung von Aminopropionitrilen nach Anspruch 1, dadurch gekennzeichnet, daß man die Umsetzung bei Drücken von 150 bis 230 bar durchführt.

7. Verfahren zur Herstellung von Aminopropionitrilen nach Anspruch 1, dadurch gekennzeichnet, daß man als Heterogenkatalysator Siliciumdioxid einsetzt.

8. Verfahren zur Herstellung von Aminopropionitrilen nach Anspruch 1, dadurch gekennzeichnet, daß man als Heterogenkatalysator Aluminiumoxid einsetzt.

9. Verfahren zur Herstellung von Aminopropionitrilen nach Anspruch 1, dadurch gekennzeichnet, daß man als Heterogenkatalysator Gemische aus Siliciumdioxid und Aluminiumoxid einsetzt.

## Claims

1. A process for the preparation of an aminopropionitrile of the formula I 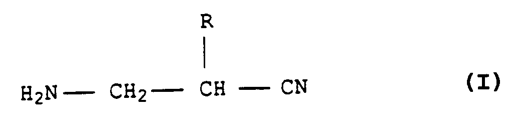 where R is hydrogen or methyl, wherein ammonia is reacted with an acrylonitrile of the formula II 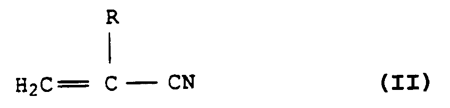 where the substituents have the abovementioned meanings, in a molar ratio of from 1 : 1 to 500 : 1 over a heterogeneous catalyst at from 40 to 180°C and from 10 to 350 bar, the catalyst being an oxide of the second or third or fourth main group or of the second to sixth subgroup of the Periodic Table of elements, an acidic zeolite, a phosphate or a heteropoly acid or a mixture thereof.

2. A process for the preparation of an aminopropionitrile as claimed in claim 1, wherein R is hydrogen.

3. A process for the preparation of an aminopropionitrile as claimed in claim 1, wherein the heterogeneous catalyst used is an acidic heterogeneous catalyst.

4. A process for the preparation of an aminopropionitrile as claimed in claim 1, wherein ammonia is used with an acrylonitrile II in a molar ratio of 10 : 1 to 80 : 1.

5. A process for the preparation of an aminopropionitrile as claimed in claim 1, wherein the reaction is carried out at from 50 to 120°C.

6. A process for the preparation of an aminopropionitrile as claimed in claim 1, wherein the reaction is carried out at from 150 to 230 bar.

7. A process for the preparation of an aminopropionitrile as claimed in claim 1, wherein the heterogeneous catalyst used is silica.

8. A process for the preparation of an aminopropionitrile as claimed in claim 1, wherein the heterogeneous catalyst used is alumina.

9. A process for the preparation of an aminopropionitrile as claimed in claim 1, wherein the heterogeneous catalyst used is a mixture of silica and alumina.

## Revendications

1. Procédé de préparation d'aminopropionitriles de la formule générale I : 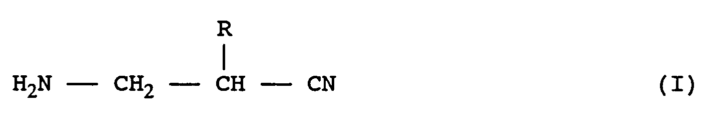 dans laquelle
R représente un atome d'hydrogène ou le radical méthyle,
caractérisé en ce que l'on fait réagir de l'ammoniac avec des acrylonitriles de la formule générale II : 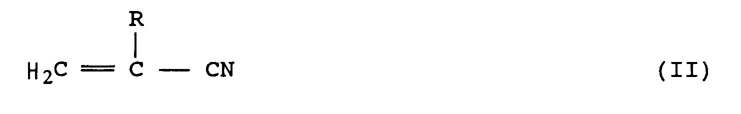 dans laquelle les substituants ont les significations qui leur ont été attribuées ci-dessus, dans le rapport molaire de 1:1 à 500:1, sur des catalyseurs hétérogènes appartenant aux oxydes du second et/ou du troisième et/ou du quatrième groupe principaux, du second au sixième sous-groupes du système périodique des éléments, des zéolites acides, des phosphates et/ou des hétéropolyacides ou des mélanges de ceux-ci, à des températures de 40 à 180°C et sous des pressions de 10 à 350 bars.

2. Procédé de préparation d'aminopropionitriles suivant la revendication 1, caractérisé en ce que R représente un atome d'hydrogène.

3. Procédé de préparation d'aminopropionitriles suivant la revendication 1, caractérisé en ce que l'on utilise des catalyseurs hétérogènes acides à titre de catalyseurs hétérogènes.

4. Procédé de préparation d'aminopropionitriles suivant la revendication 1, caractérisé en ce que l'on utilise l'ammoniac et les acrylonitriles II dans le rapport molaire de 10:1 à 80:1.

5. Procédé de préparation d'aminopropionitriles suivant la revendication 1, caractérisé en ce que l'on entreprend la réaction à des températures de 50 à 120°C.

6. Procédé de préparation d'aminopropionitriles suivant la revendication 1, caractérisé en ce que l'on entreprend la réaction sous des pressions de 150 à 230 bars.

7. Procédé de préparation d'aminopropionitriles suivant la revendication 1, caractérisé en ce que l'on utilise le dioxyde de silicium à titre de catalyseur hétérogène.

8. Procédé de préparation d'aminopropionitriles suivant la revendication 1, caractérisé en ce que l'on utilise l'oxyde d'aluminium à titre de catalyseur hétérogène.

9. Procédé de préparation d'aminopropionitriles suivant la revendication 1, caractérisé en ce que l'on utilise des mélanges constitués de dioxyde de silicium et d'oxyde d'aluminium à titre de catalyseurs hétérogènes.
